# EUROPEAN PATENT SPECIFICATION

(11) **EP 1 023 016 B1**
(45) Date of publication and mention of the grant of the patent: **13.08.2003**
(21) Application number: 97927760.5
(22) Date of filing: 28.05.1997
(51) Int. Cl.: A61F 13/00

(54) **SOLID STATE FLUID DELIVERY SYSTEM**
SYSTEM ZUM AUSTRAGEN VON FLÜSSIGKEITEN MIT CHIPS
SYSTEME TRANSISTORISE POUR DISTRIBUTION DE FLUIDES

(43) Date of publication of application: 02.08.2000
(73) Proprietor: Microdose Technologies Inc., Monmouth Jct., NJ 08852 (US)
(72) Inventor: GUMASTE, Anand V., Robbinsville, NJ 08691 (US); ABRAMS, Andrew L., Westport, CT 06880 (US); FLEMING, Scott, Ewing, NJ 08628 (US)
(74) Representative: Ilgart, Jean-Christophe
(86) International application number: US9708903
(87) International publication number: WO98053777

(56) References cited:
- US-A- 4 944 659
- US-A- 5 474 527

## Description

The present invention relates generally to fluid delivery systems and more particularly, to solid state delivery systems that do not require a needle-like nozzle and that utilize piezoelectric materials to achieve the rapid injection of accurate small quantities of fluid.

The delivery of fluids, including within the definition of this term liquids, solid particles and gases, in a variety of contexts has progressed in recent years to embrace various automated transport and delivery systems. Specifically, the development of equipment requiring rapid delivery of microquantities of fluid to a particular location or target, finds its application to such diverse areas as, for example, fuel delivery for internal combustion and turbine-type engines, the delivery of reagents in chemical and biological research and diagnostic procedures, and the delivery of microquantities of medicaments to patients for therapeutic purposes. In all of these instances, the extant technology and equipment is limited in its ability to define and control parameters for the precise delivery of exceedingly small volumes of liquid in rapid repeatable fashion. The problems and corresponding needs for improved equipment are particularly acute in the area of medicament delivery, as described in greater detail below.

Medications are injected through the skin to provide therapeutic effects which are more efficient or unobtainable through other drug delivery routes. Some reasons for injecting medications are: (1) chemical destruction of the medication by the gastrointestinal tract; (2) poor absorption; (3) patient too sick or too young to take the medicine orally; and (4) need for rapid action of the drug.

The most commonly used device to inject a medication is a hypodermic needle attached to a plunger syringe. These syringes come in a variety of sizes and can be automated by connecting them to a pump mechanism, or a pump designed for injection can be utilized. Such pumps are used primarily for intravascular or intrathecal delivery.

A number of needleless injection systems presently exist. These systems use a compressed gas, either CO₂ or compressed air. The gas is released at high pressure on demand and acts on a movable piston which forces medication out of the nozzle of the syringe. The resulting high velocity jet stream deposits the medication under the skin of a patient. All needleless injection systems have the advantage of not requiring "sharps" which are considered a bio-hazard and require careful disposal.

Present needleless injection systems are generally large and noisy. They require either the cocking of a spring mechanism or an attachment to a CO₂ source. Use of these devices requires a trained, skilled operator. Also, they must be disassembled to be cleaned. Further more, they cannot be programmed for automated delivery.

Certain devices that assist in the administration of medication and which utilize piezoelectric materials for performing certain administration functions have been described. For example, U.S. Patent Nos. 4,787,888 to Fox, 5,094,594 to Brennan, 5,415,629 to Henley, and 5,007,438 to Trachibana et al are all directed toward the administration of medications and/or the use of piezoelectric materials for such administration or otherwise.

More particularly, U.S. Patent No. 4,787,888 to Fox discloses a bandage assembly for percutaneous administration of medication wherein a piezoelectric material is utilized so as to generate sonic vibrations for assisting the medication to be absorbed through the skin of a patient. It should be noted that this patent fails to disclose the use of piezoelectric materials as injection means wherein medication is forcibly introduced through the skin of a patient.

U.S. Patent No. 5,094,594 to Brennan discloses a piezoelectric pumping device wherein piezoelectric material is utilized as a pumping means in conjunction with an electrophoretic unit. The Brennan apparatus is complex and cumbersome, and lacks the applicability to a needleless medicament injection system, and particularly such a system as is capable for forcibly introducing medication through the skin of a patient.

U.S. Patent No. 5,415,629 to Henley discloses a programmable apparatus for the transdermal delivery of medication wherein piezoelectric elements are utilized for providing ultrasonic vibrations which enhance penetration of the medication through the skin of a patient. It should be noted that this patent fails to disclose the use of piezoelectric materials as injection means wherein medication is forcibly introduced through the skin of a patient.

U.S. Patent No. 5,007,438 to Tachibana et al disclose an endermic application kit for external medicines wherein an ultrasonic oscillation is utilized to enhance the absorption of medication by the skin of a patient. It should be noted that this patent fails to disclose the use of piezoelectric materials as injection means wherein medication is forcibly introduced through the skin of a patient. In fact, this patent fails to disclose the use of piezoelectric materials in any manner.

U.S. Patent n° 5,474,527 to Bettinger discloses a microprocessor controlled transdermal medication patch system wherein said medication is dispensed internally by positive displacement from multiple reservoirs within said patch so as to vary the drug selection, sequence, and concentration and thereby the regimen and release rate. In a preferred embodiment, electric resistance heating elements activate multiple heat-shrink polymer reservoirs to dispence beneficial fluids into a common absorbent layer for transdermal passage.

U.S. Patent n° 4,944,659 to Labbe et al discloses a dispensing device for use in a implantable drug delivery system for ambulatory patients. The device comprises a pump in a drug reservoir and a piezoelectric disc element bonded to a diaphragm member forming one wall of a pump chamber and a battery and electrical circuits for cyclically applying electrical voltage to the piezoeletric member for inducing pumping movement in the diaphragm member to pump drugs from a reservoir via a valve and to a delivery catheter via another valve. A gas spring is provided to move the pump to maintain adequate pressure in the drug reservoir.

Although the above-mentioned patents are generally directed toward the metered delivery of fluids, including in some instances, medications, and illustrate prior applications of piezoelectric materials for their specific objectives, none are directed toward providing a method for utilizing piezoelectric materials as injection means wherein medication is forcibly introduced through the skin of a patient. Such a method would realize all of the benefits of a needleless injection system along with many other advantages as detailed below.

More generally, the development of a simple and inexpensive delivery system that can transfer small amounts of fluid on a rapid, quiet, and repeatable basis would be highly valued not only in regard to the injection of medicaments, but in the other commercial and industrial areas listed above where similar needs exist. Accordingly, it is toward the fulfillment of the needs expressed above that the present invention is directed.

In its broadest aspect the present invention extends to an apparatus for the rapid delivery of a fluid. The apparatus of the invention in one aspect comprises a fluid storing component comprising at least one housing having walls defining a dispensing chamber, that may be of adjustable size for containing the fluid to be dispensed, at least one nozzle element defined in at least one of said walls for the discharge therethrough of said fluid, and a second housing which, in one embodiment, may be adapted for detachable association to said first housing, including pump means for receiving and forcing a predetermined volume of said fluid out of said dispensing chamber and through said nozzle element toward said target. The pump means comprises at least one piezoelectric element adapted to cooperate with said at least one housing to impose a predetermined pressure within said dispensing chamber to force the predetermined volume of said fluid through the nozzle element and to delivery said fluid to the intended target.

An actuator for the excitation of the piezoelectric elements is included, which may include means for controlling the amount and temporal schedule for dispensing of said fluid. For example, the actuator of the invention may include a battery unit for portable application of the apparatus, and circuitry including a programmable controller for pre-setting the amount, frequency and duration of fluid dispensing. This capability has particular value in the instance where the inventive apparatus is applied to the administration of medication, whether in a clinical setting or in a constant and personal setting. In a preferred embodiment, the pump means comprises a plurality of piezoelectric elements in a stacked relationship, and adapted for sequential excitation to simulate a peristaltic motion, to receive a predetermined quantity of the fluid to be dispensed and thereafter to eject the fluid toward the intended target.

The invention also extends to piezoelectric materials as a pump means as described above, wherein the excitation of the piezoelectric element forces the fluid to egress the dispensing chamber and delivers the fluid to its intended target. More particularly, the apparatus comprises a plural its of piezoelectric elements with sequential excitation, each element associated with respective fluid chambers that are in communication with each other, to convey a predetermined amount of fluid from a reservoir to the chamber from which the fluid is finally dispensed to its intended delivery site. Alternatively, when plural piezoelectric elements are disposed in stacked adjacent relationship, the sequential excitation first defines a chamber that receives a predetermined volume of the fluid, and thereafter, ejects the fluid from the chamber by the rapid reduction in chamber volume. The application of this sequential arrangement facilitates both speed and accuracy, as precise metering and rapidly repeatable delivery are enabled.

A particular apparatus comprises a solid state needleless injection system which comprises a first housing having walls defining a dispensing chamber, a valve assembly comprising a cooperating valve stop and valve seat formed in said first housing and located in fluid communication with said dispensing chamber, and a fluid reservoir operatively connected to the first housing; and a second housing defining walls one of which is in communication with said first housing, which second housing contains at least one piezoelectric element adapted to communicate with the first housing to dispense the fluid from the system. In a particular embodiment, the first and second housings define common walls that may be detachably attached to each other, and an additional piezoelectric element is contained within the second housing and is positioned for operative communication with the valve assembly located in the second housing, to alternately permit and prevent fluid from entering said dispensing chamber from said fluid reservoir.

The fluid reservoir provides fluid medication to the dispensing chamber through the valve assembly and the fluid medication is forced out of the dispensing chamber through one or more injection ports upon actuation of the piezoelectric actuator associated with the dispensing chamber. The piezoelectric actuator interacts with the dispensing chamber by impacting the wall of the first housing that is contiguous thereto, in such a manner that the overall capacity of the dispensing chamber may be altered by altering the degree of actuation of the piezoelectric actuator. Thus, the piezoelectric actuator acts as a driver which forces the fluid medication out of the dispensing chamber whereby it is forcibly introduced through the skin of a patient.

The valve assembly comprises a valve stop that is preferably formed in the portion of the wall of the first housing that is proximate the valve seat, and as stated above, a second piezoelectric element is preferably contained within the first housing for controlling the position of the valve stop within the valve seat and hence the fluid flow between the fluid reservoir and the dispensing chamber.

The apparatus of the present invention may be constructed as a portable unit using battery operation, in the instance where it is used for the delivery of medication to an ambulatory individual. Further, the first housing including the fluid reservoir and the dispensing chamber may be fabricated as a unit that is adapted to be detached from the second housing. In a further embodiment, the second housing may be discarded after all of the fluid is expelled. The apparatus may also include programming capability, so that an extended schedule of delivery can be preset, and sufficient medication supplied, to reduce periodic inspection of the device and replenishment of the medicament.

A particular advantageous application of the inventive pump construction and apparatus is found in its use as a needleless injection means for the delivery of a fluid medication as by forcible introduction through the skin of a patient. The pump means may therefore comprise at least two piezoelectric elements disposed in stacked arrangement, with at least one opening journaled through adjacent layers. The size of the opening may be adjusted in use in response to excitation or relaxation of the piezoelectric element or elements. It can thereby be appreciated that the sequential excitation of the piezoelectric elements as described herein, can first define a chamber for the reception of a quantity of the fluid to be dispensed, and can thereafter contract the defined volume of such chamber to eject the fluid toward the intended target.

An actuator for the excitation of the piezoelectric elements is included, which may include means for controlling the amount and temporal schedule for dispensing of said fluid. For example, the actuator of the invention may include a battery unit for portable application of the apparatus, and circuitry including a programmable controller for pre-setting the amount, frequency and duration of fluid dispensing. This capability has particular value in the instance where the inventive apparatus is applied to the administration of medication, whether in a clinical setting or in a constant and personal setting.

The invention comprises an apparatus for the rapid and accurate delivery of medical reagents and medicaments, and extends to a needleless injection system which is capable of forcibly delivering medication transdermally to a patient. The apparatus comprises a needleless injection system comprising a fluid container component comprising a first housing defining a dispensing chamber and including at least one nozzle element, and a second fluid dispensing component comprising preferably a plurality of piezoelectric elements which are stacked in a layered fashion within the housing, and an actuator means including electronic circuitry and in the instance of a portable unit, a battery, which may be disposed proximate to the piezoelectric elements. More particularly, the first housing may comprise a fluid reservoir operatively connected to the second housing so as to interface with the layered stack of piezoelectric elements. The above-stated components may be modular in construction, to allow for the repeated use of the fluid dispensing component containing the piezoelectric elements and the electronic circuitry, and the disposal of the first housing or fluid container component. The first and second housings would accordingly be detachably attached to each other and may, as a unit, be releasably affixed to a surface that is to receive the fluid to be dispensed, as in the instance of the removable attachment to a patient.

As stated above, the layered stack of piezoelectric elements has at least one common aperture formed therein which extends therethrough. Each aperture may be opened and closed in each piezoelectric element relative to the degree of excitation applied, by the electronic circuitry, to each piezoelectric element. Thus, the size of each aperture in each piezoelectric element may be individually controlled by the electronic circuitry so as to allow fluid medication from the fluid reservoir to be either present or absent from the aperture area. Consequently, the layered stack of piezoelectric elements may be utilized as a pump wherein fluid medication from the fluid reservoir is forced into and out from each aperture in each piezoelectric element in the layered stack and then forcibly introduced through the skin of a patient. During this pump action, the apertures act as sealed dispensing chambers where fluid medication is temporarily stored as it flows through the layered stack of piezoelectric elements.

As noted supra, the apparatus of the present invention may be constructed as a portable unit with battery operation, in the instance where it is used for the delivery of medication to an ambulatory individual. Further, the housing including the fluid container may be fabricated either as an integral unit with the fluid dispensing component unit that may be discarded in its entirety, or as a modular construction wherein it is adapted to be detached from the second fluid dispensing component either for refilling or disposal.

The use of piezoelectric actuation to mechanically force the discrete volume of fluid rapidly and at high pressure, together with the simplified construction of the apparatus makes the manufacture and use of the present apparatus particularly attractive, and further augments the advantages thereof. For example, medications dispensed with the present apparatus may be delivered in small doses within short time intervals, and may thereby provide a more precise administration of a medication.

Accordingly, a principal object of the present invention is to provide an apparatus for the rapid and efficient delivery of fluids to target destinations, that are simple and economical to manufacture and use.

It is a further object of the present invention to provide an apparatus as aforesaid, that utilizes piezoelectric materials as a pump or injection means capable of forcibly delivering the fluid to the target destination.

It is a still further object of the present invention to provide an injection system that utilizes the properties of piezoelectric materials to respond rapidly to applied potentials, and to apply them as valves and powerful pumps and to assemble them as a unit.

Another object of the present invention is to provide a needleless, quiet, compact, inexpensive, easy-to-use, battery operated injection system, that is useful for the delivery of medication to a patient.

Another object of the present invention is to reduce medication delivery pain by many small low volume injections.

Another object of the present invention is to reduce the skill required by an operator of an injection system, and allow self-medication injection, or medication injection by persons not specially trained.

Another object of the present invention is to provide an injection system which would allow medications which are given orally, rectally, or by other means for convenient home delivery to be delivered comfortably, and to provide all the benefits of transcutaneous drug delivery.

Another object of the present invention is to have programmability, and the possibility of continuous, or predetermined interval, medication delivery by an attached system.

The invention provides a solid state needleless injection apparatus in accordance with claim 1 and the dependent claims.

Other objects and advantages of the present invention will become apparent to those skilled in the art from a review of the following detailed description and claims, in conjunction with the accompanying drawings which are appended thereto.

In order to facilitate a fuller understanding of the present invention, reference is now made to the appended drawings. The drawings should not be construed as limiting the present invention, but are intended to be exemplary only.
Figure 1 is a functional block diagram of a solid state fluid delivery system according to a particular embodiment of the present invention;
Figure 2 is a functional block diagram of the delivery system shown in Figure 1 with an alternate fluid reservoir;
Figure 3 is a functional block diagram of the delivery system shown in Figure 2 with an alternate second housing structure;
Figure 4 is a cross-sectional view of a needleless injection system according to the present invention.
Figure 5 is a cross-sectional view of some of the components of the needleless injection system shown in Figure 4 along with another type of fluid reservoir and Figure 5a is a detailed view of the layered stack of piezoelectric elements.
Figure 6 is a close-up cross-sectional view of a common aperture in the layered stack of piezoelectric elements according to the present invention, along with a functional representation of a fluid reservoir.
Figures 7a-7d are enlarged cross-sectional views showing the sequence of operation of a needleless injection system according to the present invention.

In its broadest aspect, the present invention relates to a delivery system for fluids, including powders, liquids and gases, that is capable of rapidly repetitive discharge of a precise and minute amount of such fluid on a continuous basis. Such a system finds particular application in a variety of contemporary industrial settings, extending from machinery design and operation, to medical devices for the administration of reagents and medications.

Accordingly, the apparatus of the invention comprises: at least one first housing having walls defining a dispensing chamber of adjustable volume for said fluid, and at least one nozzle element defined in one of said walls for the discharge of said fluid; at least one second housing which may be adapted, in one embodiment, for detachable association with said first housing, including pump means comprising at least one piezoelectric element for forcing a predetermined quantity of said fluid out of said dispensing chamber and through said at least one nozzle element; and actuation means including controller means for exciting said pump means to force said fluid out of said dispensing chamber, and for controlling the operation of said pump means.

A particular embodiment of the contemplated apparatus is illustrated herein in relation to an apparatus for the needleless delivery of medication, and the following description is presented in detailed exposition of that application and embodiment. It is to be understood, however, that the features and principles of the invention extend beyond the following non-limiting illustration.

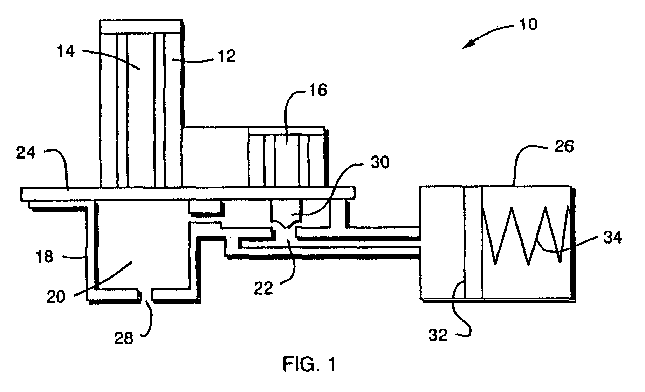

Referring to the figures wherein like numerals designate like parts, and particularly to Figure 1, there is shown a solid state needleless injection system 10 according to the present invention that utilizes piezoelectric materials so as to provide a force that is capable of injecting matter contained within a dispensing chamber through the skin of a patient. The solid state needleless injection system 10 comprises a second housing structure 12 containing a first piezoelectric element 14 and a second piezoelectric element 16, both elements 14 and 16 functioning as actuators of pumping movement; and a first housing structure 18 defining a dispensing chamber 20 of adjustable volume. Such volume adjustment may be accomplished by means of a deformable wall 24 which may be a flexible membrane, or by the disposition within chamber 20 of a wall having inward spring bias, such as that illustrated with reference to fluid reservoir 26, described below. Accordingly, the invention should not be limited to the specific adjustment capability and means illustrated herein.

Housing 18 includes a valve assembly comprising a valve seat 22, and a fluid reservoir 26 operatively connected to housing 18. The dispensing chamber 20, and hence the second housing structure 18, has at least one injection port 28 formed therein near the bottom thereof. The valve assembly also includes a valve stop 30 associated with deformable wall 24 as illustrated and located proximate to valve seat 22 and the second piezoelectric element 16. The fluid reservoir 26 has a plunger 32, and a biasing member, such as a piezoelectric element, or a spring 34 as illustrated, may be disposed therein to force fluids such as fluid medications that are contained therein into housing structure 18.

In a particular embodiment, the solid state needleless injection system 10 comprises two main parts: (1) the second housing structure 12 containing the first piezoelectric actuator 14 and the second piezoelectric actuator 16, which is reusable; and (2) a disposable cassette-like unit comprising the first housing structure 18 as described above, including fluid reservoir 26.

In operation, the second piezoelectric element 16, and hence the valve stop 30, is first retracted so as to allow fluid in the fluid reservoir 26 to flow through the valve seat and into the dispensing chamber. The first piezoelectric element 14 is then retracted so that the dispensing chamber 20 is totally filled. Next, the second piezoelectric element 16 is excited so that the valve stop 30 seals the valve seat 22 thereby stopping the fluid from flowing in either direction between the fluid reservoir 26 and the dispensing chamber 20. The first piezoelectric element 14 is then excited so that the volume of the dispensing camber 20 is rapidly decreased and the fluid contained therein is displaced at high pressure. Thus, the fluid is forced out of the dispensing chamber 20 through the injection port 28 at a pressure sufficient in the instance of a medication, to penetrate the skin of a patient. This cycle can be repeated at various rates, but it is preferably repeated within the 100 to 2000 Hz range.

As previously mentioned, more than one injection port 28 can be provided in the dispensing chamber 20, and hence the first housing structure 18. Such would allow for a more widespread injection area which would reduce discomfort and irritation at an injection site. It should be noted that each injection port 28 is typically 0.001 inches in diameter.

Both the second housing structure 12 and the first housing structure 18 may be fabricated of a variety of rigid materials, such as plastic or metal. In the instance where the fluid chamber 20 is compressible, a deformable wall 24 is included that may be fabricated of a variety of flexible materials, such as clear silicon. It should be further noted that the valve stop 30 may be separate from the wall 24.

Both the first piezoelectric element 14 and the second piezoelectric element 16 may be fabricated from a variety of piezoelectric materials, such as lead-zirconate/lead titanate (PZT). It should be noted, however, that the second piezoelectric actuator 16 may be replaced with a typical electromechanical actuator (not shown) comprising a magnet and a coil.

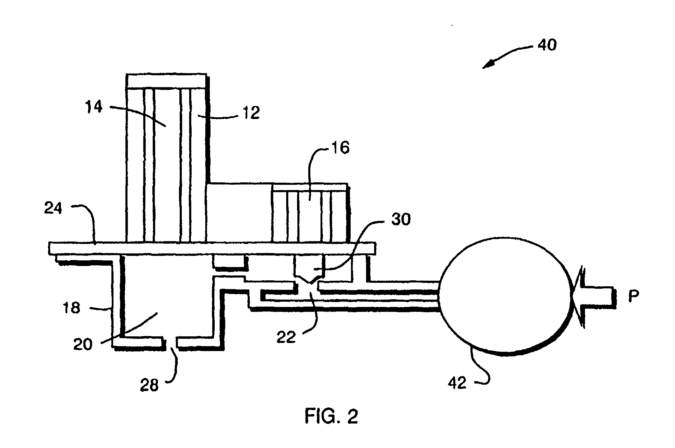

The fluid reservoir 26 may be fabricated of a variety of materials, such as plastic. Also the fluid reservoir 26 may take many different forms. For instance, referring to Figure 2, there is shown a second embodiment of a solid state needleless injection system 40 having a fluid reservoir 42 that may be fabricated of a collapsible material. In such a case, external pressure P may be applied thereto so as to force the fluid medication contained therein into the second housing structure 18.

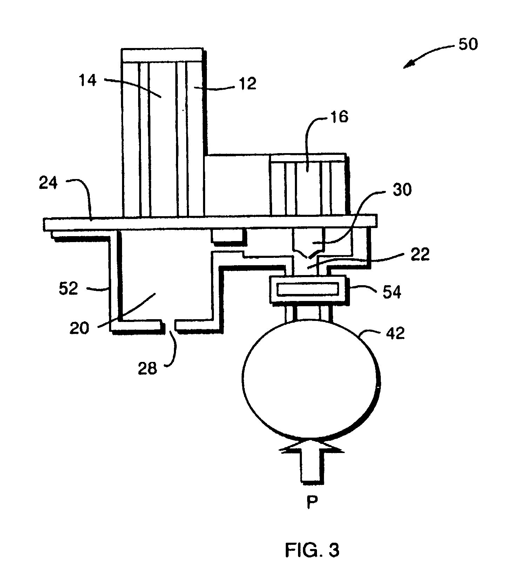

The second housing structure 18 may also take many different forms. For instance, referring to Figure 3, there is shown a third embodiment of a solid state needleless injection system 50 having a first housing structure 52 that has a luer-lock interface 54 formed therein for connection with the fluid reservoir 42. Such an interface 54 allows for quick and easy attachment and removal of the fluid reservoir 42 from the first housing structure 52.

Throughout all of the above-described embodiments, an external electrical excitation (not shown) is required for the first piezoelectric element 14, the second piezoelectric element 16, and the electromechanical actuator (not shown). Such external electrical excitation typically comprises a DC voltage source having electrodes which are attached to the piezoelectric materials and to the coil, respectively.

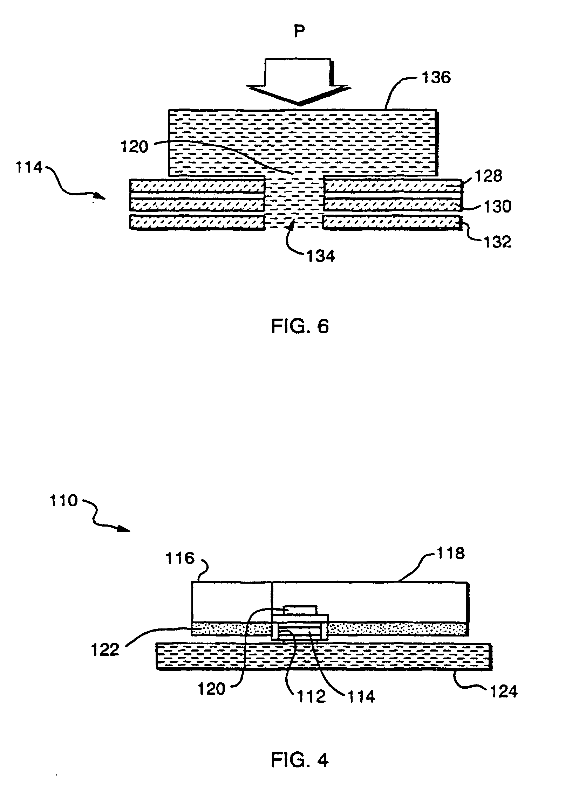

Referring to Figure 4, there is shown a solid state needleless injection system 110 according to another embodiment of the present invention that utilizes piezoelectric materials so as to provide a force that is capable of injecting matter contained within a sealed dispensing chamber through the skin of a patient. The needleless injection system 110 comprises a second housing structure 112, plural piezoelectric elements 114 (three illustrated herein) stacked in a layered fashion within second housing structure 112, electronic circuitry 116 disposed proximate second housing structure 112 and hence the layered stack of piezoelectric elements 114; and a first housing structure comprising a fluid reservoir 118, disposed proximate to a second housing 112 and operatively connected thereto so as to allow fluid medication contained with the fluid reservoir 118 to communicate with the layered stack of piezoelectric elements 114. The interface 120 between the fluid reservoir 118 and the second housing structure 112 as illustrated, may comprise a luer-lock connector which allows for quick and easy attachment and removal of the fluid reservoir 118 from housing structure 112. While the preceding description relates to the use of a modular design where the fluid reservoir 118 and the second housing structure 112 are separable, the economies of manufacturing cost allow for the fabrication of the present apparatus as a single unit that, for example, may be disposable in its entirety. Accordingly, the present invention is intended to cover this variation within its scope.

It should be noted that the electronic circuitry 116 is provided and distributed so as to allow the piezoelectric elements 114 to be individually excited, typically with a steady state DC voltage. It should also be noted that although the use of three piezoelectric elements 114 is illustrated, the apparatus could be operated with as few as two such elements 114. Conversely, multiple units exceeding those referenced or illustrated are contemplated within the scope hereof.

Referring further to Figure 4, an adhesive base 122 may be provided upon which the second housing structure including the electronic circuitry 116 and the fluid reservoir 118, and through which the first housing structure 112 and hence the layered stack of piezoelectric elements 114 are mounted. The adhesive base 122 allows the entire needleless injection system 110 to be easily removably attached to vicinity of the target for the dispensing of fluid, such as the skin 124 of a patient illustrated herein. In the illustrated instance, the system 110 is constructed and applied in similar fashion to a bandage or a patch.

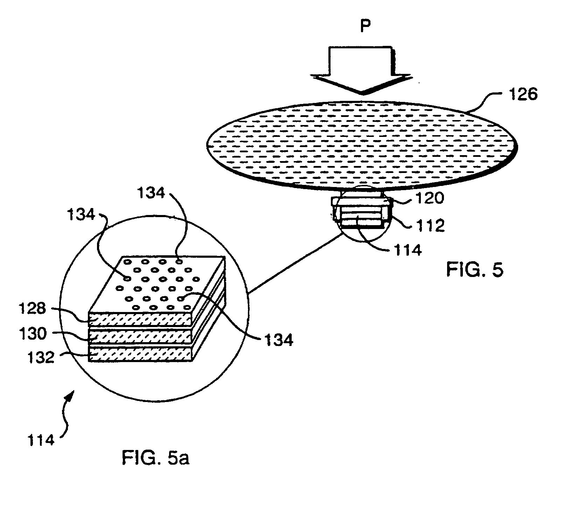

Referring to Figure 5, the second housing structure 112 and the layered stack of piezoelectric elements 114 are shown connected, through interface 120, to another type of fluid reservoir 126. This type of fluid reservoir 126 requires external pressure to be applied thereto so as to force fluid medication therefrom, as compared to the fluid reservoir 118 described above which may have electronically actuated means for forcing fluid medication therefrom. Naturally, the fluid reservoirs illustrated herein are presented for purposes of illustration and not limitation, as the invention contemplates the use of reservoirs having various adaptations and means for the discharge or release of contained fluid.

Also shown in Figure 5 is a detailed view of the layered stack of piezoelectric elements 114. From this view, it can be seen that the layered stack of piezoelectric elements 114 as illustrated comprises an upper piezoelectric element 128, a middle piezoelectric element 130, and a lower piezoelectric element 132. As discussed earlier herein, the invention contemplates the inclusion of only two piezoelectric elements, in which event, for example, only upper element 128 and middle element 130 would be present, and would operate as described later on herein. The invention is thereby contemplated to embrace this further embodiment within its scope.

Referring further to Figures 5 and 5a, piezoelectric elements 128, 130 and 132 as illustrated are secured to each other with an adhesive (not shown). Of course, elements 128 may clamp together, and the invention is not limited to a particular method of securement. Should an adhesive be used, such adhesive may be of a type that will not allow fluid medication to seep therethrough. Also, it should be noted that such an adhesive should desirably be pliable so as to allow the individual piezoelectric elements 128, 130 and 132 to move relative to one another, as will be described in detail below. Further, although the layered stack of piezoelectric elements 114 is shown to be rectangular in shape, stack 114 may be formed in a variety of shapes such as, for example, as a cylinder. Likewise, the shape of the layered stack of piezoelectric elements 114 and the shape of the rigid housing structure 112 may correspond so as to ensure efficient and compact construction of this component of the system 110.

A plurality of common apertures 134 are formed in the layered stack of piezoelectric elements 114. The apertures 134 are termed common because they are aligned and extend down through the entire layered stack of piezoelectric elements 114. The diameter of each of the apertures 134 may vary, and for example, may be as small as 150 micrometers. In such last-mentioned instance, the apertures 134 may be preferably formed using a laser.

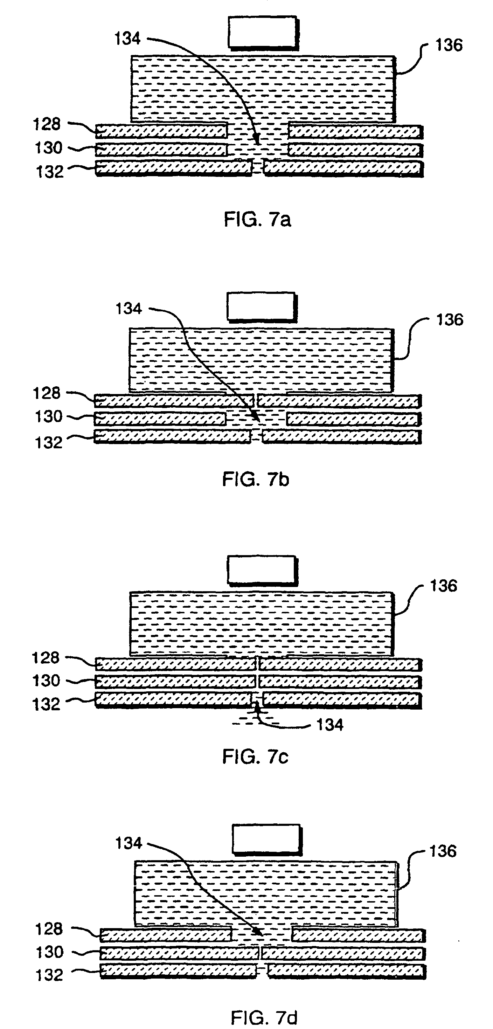

Referring to Figure 6, the layered stack of piezoelectric elements 114 is shown with a common aperture 134, along with a functional representation of a fluid reservoir 136 and an interface 120. In this particular view, all of the piezoelectric elements 128, 130 and 132 are in a dormant state (i.e. not excited). Thus, the common aperture 134 has a constant diameter extending all the way through the layered stack of piezoelectric elements 114.

Referring to Figure 7a, the lower piezoelectric element 132 is excited so as to narrow its section of the common aperture 134 to about 25 microns in diameter. This step allows fluid medication to enter into the aperture 134 adjacent the upper piezoelectric element 128 and the middle piezoelectric element 130. In the instance where lower piezoelectric element 132 is absent, the aperture 134 could be provided with a constricted orifice or nozzle element that would effectively curtail appreciable fluid escape and thereby confer an equivalent function and effect to that of piezoelectric element 132.

Referring to Figure 7b, the upper piezoelectric element 128 is excited so as to narrow its section of the common aperture 134. This step essentially creates a sealed dispensing chamber in the aperture 134 adjacent the middle piezoelectric element 130.

Referring to Figure 7c, the middle piezoelectric element 130 is excited so as to narrow its section of the common aperture 134. This step forces the fluid medication out of the sealed dispensing chamber (i.e. the second of the common aperture 134 adjacent the middle piezoelectric element 130) and through the aperture opening in the lower piezoelectric element 132. Since, in application, the lower piezoelectric element 132 will be directly abutting the skin of a patient, the fluid medication will be forcibly introduced through the skin of the patient.

Referring to Figure 7d, the upper piezoelectric element 128 is relaxed so as to expand its section of the common aperture 134 and thereby allow the entire sequential process to be repeated. Indeed, this process can be repeated until all of the fluid medication is dispensed from a fluid reservoir 118, 126. This entire sequential process can probably best be described as a peristaltic pumping action.

First housing structure 112 may be fabricated of a variety of materials, such as plastic or metal, and second housing structure including fluid reservoir 118, 126 may be fabricated of a variety of materials, such as plastic or a collapsible material. Also, fluid reservoir 118, 126 may take many different forms as indicated in Figures 4 and 5. Further, piezoelectric elements such as illustrative elements 128, 130, and 132 may be fabricated from a variety of piezoelectric materials, such as lead-zirconate/lead titanate (PZT).

Finally, it should be noted that throughout all of the above-described embodiment, electrical connections must be made between the electronic circuitry 116 and the individual piezoelectric elements 128, 130 and 132. This is typically accomplished by providing each of the piezoelectric elements 128, 130 and 132 with electrodes (not shown) and electrically connecting these electrodes to the electronic circuitry 116. The electronic circuitry 116 may be somewhat sophisticated so as to allow programmability, with the possibility of continuous, or predetermined interval, delivery of the fluid medication.

Thus, the fluid medication is forced out of the dispensing chamber 120 through the injection port 128 at a pressure sufficient enough to penetrate the skin of a patient. This cycle can be repeated at various rates, and an illustrative, non-limiting range of repetition of from about 100 to about 2000 Hz range may be used.

As previously mentioned, more than one injection port 128 can be provided in the dispensing chamber 120, and hence in the second housing structure 118. Such would allow for a more widespread injection area which would reduce discomfort and irritation at an injection site. An illustrative and non-limiting size for an injection port 28 may be about 0.0025 cm. in diameter.

Throughout all of the above-described embodiments, an external electrical excitation (not shown) is required for the piezoelectric elements 128, 130 and 132. Such external electrical excitation typically comprises a DC voltage source having electrodes which are attached to the piezoelectric materials and to the coil, respectively.

With the present invention method now fully described, it can thus be seen that the primary objective set forth above is efficiently attained, and since certain changes may be made in the above-described embodiments without departing from the scope of the invention, it is intended that all matter contained in the above description or shown in the accompanying drawings shall be interpreted as illustrative and not restrictive, the scope of the invention being defined in the appended claims.

## Claims

1. A solid state needleless injection apparatus for the rapid and repeatable delivery of small quantities of a fluid medication through the skin of a patient comprising:
(a) at least one first housing (18, 118) having walls defining a dispensing chamber for said fluid, and at least one nozzle element defined in one of said walls for the discharge of said fluid; and
(b) at least one second housing (12, 112) communicating with said first housing, said second housing comprising pump means comprising at least one piezoelectric element (14, 16, 114) in fluid communication with said first housing for forcing a predetermined quantity of said fluid out of said dispensing chamber and through said at least one nozzle element, said second housing being detachable from said first housings, and actuation means for exciting said pump means to force said fluid out of said dispensing chamber.

2. The apparatus according to claim 1, **characterized in that** said first housing includes a fluid reservoir (26) for holding the quantity of fluid to be dispensed in fluid communication with said dispensing chamber, and a valve assembly (22,30) for controlling the quantity and rate of transfer of fluid from said fluid reservoir to said dispensing chamber.

3. The apparatus according to claim 1 or 2, **characterized by** further including a secondary pump means (34) associated with said fluid reservoir to place pressure on said reservoir to expel fluid contained therein.

4. The apparatus according to claim 1, **characterized in that**
said first housing (18) has at least one movable wall (32) defining a dispensing chamber of variable volume formed therein; and
said at least one piezoelectric actuator (14) as disposed in communication with a wall of said first housing and being excitable so as to provide a driving force against said wall to thereby force fluid from within said dispensing chamber through an output port (28).

5. The apparatus according to claim 4, **characterized in that**
said dispensing chamber also has an input port formed therein, wherein said input port allows fluid to enter into said dispensing chamber.

6. The apparatus according to claim 5, **characterized in that** said fluid reservoir has an output port formed therein in fluid communication with said input port of said dispensing chamber so as to allow said fluid to flow from said fluid reservoir to said dispensing chamber.

7. The apparatus according to claim 5, **characterized by** including a valve assembly (22, 30) operatively connected between said output port of said fluid reservoir and said input port of said dispensing chamber so as to control the flow of fluid between said fluid reservoir to said dispensing chamber.

8. The apparatus according to claim 5, **characterized by** including a valve assembly comprising:
a valve seat (22) formed in said first housing;
a valve stop (30) formed in said first housing and positioned to cooperate with said valve seat to alternately permit and prevent the flow of fluid from said fluid reservoir and to said dispensing chamber; and
an actuation means (16) for controlling the position of said valve stop with respect to said valve seat.

9. The apparatus according to any of claims 1-8, **characterized by** comprising a plurality of piezoelectric elements (114) disposed in a stacked arrangement adjacent one another, each of said piezoelectric elements having at least one opening therein, said openings being journaled within each of said piezoelectric elements in axial alignment between adjacent said piezoelectric elements, said axially aligned stacked piezoelectric elements being pliably sealed to one another whereby to define a non-porous chamber for receiving and holding a predetermined volume of said fluid, said plurality of piezoelectric elements being adapted for sequential excitation to simulate a peristaltic motion whereby to receive a predetermined volume of said fluid from said dispensing chamber, and thereafter to eject the same through said at least one nozzle element toward said target; and actuation means (116) for exciting said piezoelectric activators.

10. An apparatus according to claim 9, **characterized in that** said activating means (116) comprises:
electronic circuitry electrically connected to said layered stack of piezoelectric elements so as to allow each of said plurality of piezoelectric elements to be individually excited and the size of each of said aperture to be individually controlled, thereby allowing said layered stack of piezoelectric elements to be utilized as a pump wherein fluid medication from said fluid reservoir may be forced into and out from each said aperture and ultimately be forcibly introduced through the skin of a patient.

11. The apparatus according to claim 1, **characterized in that** each of said plurality of piezoelectric elements has a plurality of apertures (134) formed therein, wherein said plurality apertures are aligned in groups so as to form a plurality of common apertures extending through said layered stack of piezoelectric elements from said first opening to said second opening.

## Patentansprüche

1. Nadellose Festkörperinjektionsvorrichtung für die schnelle und wiederholbare Verabreichung kleiner Mengen einer fluidförmigen Arznei durch die Haut eines Patienten, die umfaßt:
(a) wenigstens ein erstes Gehäuse (18, 118) mit Wänden, die eine Abgabekammer für das Fluid bilden, und wenigstens einem Düsenelement, das in einer der Wände für die Abgabe des Fluids definiert ist; und
(b) wenigstens ein zweites Gehäuse (12, 112), das mit dem ersten Gehäuse in Verbindung steht und eine Pumpeinrichtung mit wenigstens einem piezoelektrischen Element (14, 16, 114), das mit dem ersten Gehäuse in einer Fluidverbindung steht, um eine vorgegebene Menge des Fluids aus der Abgabekammer und durch das wenigstens eine Düsenelement zu zwingen, wobei das zweite Gehäuse von den ersten Gehäusen ablösbar ist, sowie eine Betätigungseinrichtung zum Erregen der Pumpeinrichtung, um das Fluid aus der Abgabekammer zu zwingen, umfaßt.

2. Vorrichtung nach Anspruch 1, **dadurch gekennzeichnet, daß** das erste Gehäuse einen Fluidvorratsbehälter (26) zum Halten der abzugebenden Fluidmenge, der mit der Abgabekammer in einer Fluidverbindung steht, sowie eine Ventilbaueinheit (22, 30), die die Menge und den Durchfluß des Fluids von dem Fluidvorratsbehälter zu der Abgabekammer steuert, umfaßt.

3. Vorrichtung nach Anspruch 1 oder 2, ferner **gekennzeichnet durch** eine zweite Pumpeinrichtung (34), die dem Fluidvorratsbehälter zugeordnet ist, um ihn mit Druck zu beaufschlagen, um darin enthaltenes Fluid auszustoßen.

4. Vorrichtung nach Anspruch 1, **dadurch gekennzeichnet, daß**
das erste Gehäuse (18) wenigstens eine bewegliche Wand (32) besitzt, die eine darin ausgebildete Abgabekammer mit veränderlichem Volumen definiert; und
der wenigstens eine piezoelektrische Aktuator (14), der so angeordnet ist, daß er mit einer Wand des ersten Gehäuses in Verbindung steht, erregt werden kann, um auf die Wand eine Antriebskraft auszuüben, um dadurch Fluid von innerhalb der Abgabekammer durch einen Ausgangsanschluß (28) zu zwingen.

5. Vorrichtung nach Anspruch 4, **dadurch gekennzeichnet, daß**
die Abgabekammer außerdem einen in ihr ausgebildeten Eingangsanschluß besitzt, der einem Fluid ermöglicht, in die Abgabekammer einzutreten.

6. Vorrichtung nach Anspruch 5, **dadurch gekennzeichnet, daß** der Fluidvorratsbehälter einen darin ausgebildeten Ausgangsanschluß besitzt, der mit dem Eingangsanschluß der Abgabekammer in einer Fluidverbindung steht, damit das Fluid von dem Fluidvorratsbehälter in die Abgabekammer strömen kann.

7. Vorrichtung nach Anspruch 5, **gekennzeichnet durch** eine Ventilbaueinheit (22, 30), die funktional zwischen den Ausgangsanschluß des Fluidvorratsbehälters und den Eingangsanschluß der Abgabekammer geschaltet ist, um die Strömung des Fluids zwischen dem Fluidvorratsbehälter und der Abgabekammer zu steuern.

8. Vorrichtung nach Anspruch 5, **gekennzeichnet durch** eine Ventilbaueinheit, die umfaßt:
einen Ventilsitz (22), der im ersten Gehäuse ausgebildet ist;
einen Ventilanschlag (30), der in dem ersten Gehäuse ausgebildet und so positioniert ist, daß er mit dem Ventilsitz zusammenwirkt, um abwechselnd eine Strömung von Fluid von dem Fluidvorratsbehälter zu der Abgabekammer zuzulassen und zu sperren; und
eine Betätigungseinrichtung (16) zum Steuern der Position des Ventilanschlags in bezug auf den Ventilsitz.

9. Vorrichtung nach einem der Ansprüche 1-8, **gekennzeichnet durch** mehrere piezoelektrische Elemente (114), die in einer Stapelanordnung nebeneinander angeordnet sind, wobei jedes der piezoelektrischen Elemente wenigstens eine Öffnung besitzt, wobei die Öffnungen in jedem der piezoelektrischen Elemente zwischen benachbarten der piezoelektrischen Elemente axial ausgerichtet angeordnet sind, wobei die axial ausgerichteten, gestapelten piezoelektrischen Elemente biegsam gegeneinander abgedichtet sind, wodurch eine nicht poröse Kammer für die Aufnahme und zum Halten eines vorgegebenen Volumens des Fluids definiert wird, wobei die mehreren piezoelektrischen Elemente für eine aufeinanderfolgende Erregung ausgelegt sind, um eine peristaltische Bewegung zu simulieren, wodurch ein vorgegebenes Volumen des Fluids von der Abgabekammer empfangen wird und danach dieses Volumen **durch** das wenigstens eine Düsenelement zu dem Ziel ausgespritzt wird; und eine Betätigungseinrichtung (116), die die piezoelektrischen Aktuatoren erregt.

10. Vorrichtung nach Anspruch 9, **dadurch gekennzeichnet, daß** die Aktivierungseinrichtung (116) umfaßt:
eine elektronische Schaltungsanordnung, die mit dem in Lagen angeordneten Stapel aus piezoelektrischen Elementen elektrisch verbunden ist, um so zu ermöglichen, daß jedes der mehreren piezoelektrischen Elemente einzeln erregt wird, wobei die Größe jeder der Öffnungen einzeln gesteuert wird, um dadurch zu ermöglichen, daß der in Lagen angeordnete Stapel von piezoelektrischen Elementen als eine Pumpe verwendet wird, wobei die Fluidverabreichung von dem Fluidvorratsbehälter in die und aus der Öffnung gezwungen werden kann und schließlich zwangsläufig durch die Haut eines Patienten eingeführt werden kann.

11. Vorrichtung nach Anspruch 1, **dadurch gekennzeichnet, daß** jedes der mehreren piezoelektrischen Elemente mehrere darin ausgebildete Blenden (134) besitzt, wobei die mehreren Blenden in Gruppen ausgerichtet sind, um so mehrere gemeinsame Blenden zu bilden, die sich durch den aus Lagen gebildeten Stapel von piezoelektrischen Elementen von der ersten Öffnung zu der zweiten Öffnung erstrecken.

## Revendications

1. Dispositif d'injection sans aiguille à l'état solide pour la fourniture rapide et répétable de petites quantités de médicament fluide à travers la peau d'un patient, comprenant :
(a) au moins un premier logement (18, 118) ayant des parois qui définissent une chambre de distribution pour ledit fluide, et au moins un élément d'embout défini dans l'une desdites parois pour l'évacuation dudit fluide ; et
(b) au moins un second logement (12, 112) qui communique avec ledit premier logement, ledit second logement comprenant des moyens de pompage qui comportent au moins un élément piézoélectrique (14, 16, 114) en communication de fluide avec ledit premier logement pour faire sortir une quantité prédéterminée dudit fluide de ladite chambre de distribution et à travers ledit au moins un élément d'embout, ledit second logement étant amovible dudit premier logement, et des moyens d'activation pour exciter lesdits moyens de pompage pour faire sortir ledit fluide de ladite chambre de distribution.

2. Dispositif selon la revendication 1, **caractérisé en ce que** ledit premier logement comprend un réservoir de fluide (26) pour contenir la quantité de fluide qui doit être distribuée en communication de fluide avec ladite chambre de distribution, et un ensemble formant clapet (22, 30) pour contrôler la quantité et la vitesse de transfert du fluide à partir dudit réservoir de fluide vers ladite chambre de distribution.

3. Dispositif selon la revendication 1 ou 2, **caractérisé en ce qu'**il comprend également des seconds moyens de pompage (34) associés audit réservoir de fluide pour mettre la pression sur ledit réservoir pour expulser le fluide qui y est contenu.

4. Dispositif selon la revendication 1, **caractérisé en ce que** ledit premier logement (18) comprend au moins une paroi mobile (32) qui définit une chambre de distribution de volume variable qui y est formée ; et
ledit au moins un actuateur piézoélectrique (14) placé en communication avec une paroi dudit premier logement, et étant excitable de façon à assurer une force de poussée contre ladite paroi pour pousser ainsi le fluide hors de ladite chambre de distribution au travers d'un orifice de sortie (28).

5. Dispositif selon la revendication 4, **caractérisé en ce que** ladite chambre de distribution comprend également un orifice d'entrée, dans lequel ledit orifice d'entrée permet au fluide de pénétrer dans ladite chambre de distribution.

6. Dispositif selon la revendication 5, **caractérisé en ce que** ledit réservoir de fluide comprend un orifice de sortie en communication de fluide avec ledit orifice d'entrée de ladite chambre de distribution de façon à permettre audit fluide de s'écouler depuis ledit réservoir de fluide vers ladite chambre de distribution.

7. Dispositif selon la revendication 5, **caractérisé en ce qu'**il comprend un ensemble formant clapet (22, 30) reliée utilement entre ledit orifice de sortie dudit réservoir de fluide et ledit orifice d'entrée de ladite chambre de distribution de façon à contrôler le débit du fluide entre ledit réservoir de fluide et ladite chambre de distribution.

8. Dispositif selon la revendication 5, **caractérisé en ce qu'**il comprend un ensemble formant clapet comprenant :
un siège de clapet (22) formé dans ledit premier logement ;
une butée de clapet (30) formée dans ledit premier logement et positionnée pour s'accoupler avec ledit siège de clapet pour permettre et empêcher alternativement le débit de fluide à partir dudit réservoir de fluide et vers ladite chambre de distribution ; et
des moyens d'activation (16) pour contrôler la position de ladite butée de clapet par rapport audit siège de clapet.

9. Dispositif selon l'une quelconque des revendications 1 à 8, **caractérisé en ce qu'**il comprend une pluralité d'éléments piézoélectriques (114) disposés en pile adjacents les uns aux autres, chacun desdits éléments piézoélectriques comprenant au moins une ouverture, lesdites ouvertures étant en palier à l'intérieur de chacun desdits éléments piézoélectriques en alignement axial entre lesdits éléments piézoélectriques adjacents, lesdits éléments piézoélectriques empilés alignés axialement étant fixés de façon souple les uns aux autres de façon à définir une chambre non poreuse pour accueillir et contenir un volume prédéterminé dudit fluide, ladite pluralité d'éléments piézoélectriques étant adaptée à une excitation séquentielle pour simuler un mouvement péristaltique de façon à recevoir un volume prédéterminé dudit fluide depuis ladite chambre de distribution, puis expulser celui-ci à travers ledit au moins un élément d'embout vers ladite cible ; et des moyens d'activation (116) pour exciter lesdits activateurs piézoélectriques.

10. Dispositif selon la revendication 9, **caractérisé en ce que** lesdits moyens d'activation (116) comprennent :
un circuit électronique relié électriquement à ladite pile stratifiée d'éléments piézoélectriques de façon à permettre à chacun de ladite pluralité d'éléments piézoélectriques d'être excité individuellement et la taille de chacune desdites ouvertures d'être individuellement contrôlée, permettant ainsi à ladite pile d'éléments piézoélectriques stratifiée d'être utilisée comme une pompe dans laquelle le médicament fluide en provenance dudit réservoir fluide peut être poussé à l'intérieur et à l'extérieur de ladite ouverture et être finalement introduit de force à travers la peau d'un patient.

11. Dispositif selon la revendication 1, **caractérisé en ce que** chacun de ladite pluralité d'éléments piézoélectriques comprend une pluralité d'ouvertures (134) formées dans ceux-ci, dans lequel ladite pluralité d'ouvertures est alignée en groupes de façon à former une pluralité d'ouvertures communes qui s'étendent à travers ladite pile stratifiée d'éléments piézoélectriques à partir de ladite première ouverture vers ladite seconde ouverture.
